# EUROPÄISCHE PATENTSCHRIFT

(11) **EP 0 950 395 B1**
(45) Veröffentlichungstag und Bekanntmachung des Hinweises auf die Patenterteilung: **21.02.2001**
(21) Anmeldenummer: 99105201.0
(22) Anmeldetag: 12.03.1999
(51) Int. Cl.: A61K 7/13, A61K 7/00

(54) **Verfahren zur Herstellung von stabilen wässrigen Haarfärbeemulsionen**
Method for manufacturing stable aqueous hair dye emulsions
Procédé de fabrication d'émulsions aqueuses stables pour la teinture des cheveux

(30) Priorität: 06.04.1998 DE 19815338
(43) Veröffentlichungstag der Anmeldung: 20.10.1999
(73) Patentinhaber: GOLDWELL GmbH, D-64297 Darmstadt (DE)
(72) Erfinder: Golinski, Frank Dr., 64297 Darmstadt (DE)

(56) Entgegenhaltungen:
- EP-A- 0 823 250
- US-A- 5 693 317
- DATABASE WPI Derwent Publications Ltd., London, GB; AN 97-347353 XP002112429 AJINOMOTO: "Preparation of emulsified cosmetic materials e.g. shampoo - comprises mixing and emulsifying oil phase containing dissolved cationic surfactant and aqueous phase containing anionic surfactant" & JP 09 143027 A (AJINOMOTO), 3. Juni 1997 (1997-06-03)

## Beschreibung

Die vorliegende Erfindung betrifft ein verbessertes Verfahren zur Herstellung von stabilen wäßrigen Haarfärbeemulsionen.

Permanente Haarfärbemittel, die üblicherweise mindestens ein Oxidationsfarbstoff-Vorprodukt, nämlich ein Entwickler-Kuppler-System, enthalten, werden zumeist in Form wäßriger Emulsionen eingesetzt (vgl. K. Schrader, Grundlagen und Rezepturen der Kosmetika, 2. Aufl. (1989), S. 797 ff).

Deren Herstellung geschieht durch Heißemulgieren der Bestandteile und darauffolgendes Abkühlen, was naturgemäß eine längere Zeit in Anspruch nimmt und auch nicht immer zu stabilen Emulsionen führt.

Es bestand daher ein Bedürfnis, den gegenwärtigen Herstellungsprozeß zu ändern und dabei zu optimieren.

Es wurde nunmehr gefunden, daß eine stabile wäßrige Haarfärbeemulsion, die mindestens ein Oxidations-Haarfarbstoffvorprodukt enthält, mit guten rheologischen Eigenschaften unter wesentlicher Reduzierung der Herstellungszeit und entsprechender Energieersparnis dadurch hergestellt werden kann, daß zunächst eine homogene Ölphase durch Vermischen der Ölbestandteile mit mindestens einem öllöslichen Emulgator bei 40 bis 80, vorzugsweise 45 bis 70°C, hergestellt, und diese Ölphase anschließend mit einer wäßrigen Phase, die mindestens einen wasserlöslichen Emulgator enthält, unter Scherkraft, d.h. Rühren, bei 15 bis 30°C, d.h. vorzugsweise Raumtemperatur bei etwa 20 bis 25°C, vermischt wird.

Dabei weist das erhaltene Endprodukt vorzugsweise eine Viskosität zwischen 2500 und 25 000, insbesondere 5 000 und 20 000, besonders bevorzugt etwa 12 000 bis 18 000 mPa.s, gemessen bei 20°C im Brookfield-Viskosimeter RVT, auf.

Der Anteil der Ölphase in der Gesamtemulsion liegt vorzugsweise bei etwa 10 bis etwa 40, vorzugsweise 15 bis 30 Gew.-% der Gesamtemulsion.

Als Ölkörper in der Ölphase werden die üblich kosmetischen Öle und Fette, beispielsweise natürliche Öle wie Avocadoöl, Cocosöl, Palmöl, Sesamöl, Erdnußöl, Spermöl, Sonnenblumenöl, Mandelöl, Pfirsichkernöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline eingesetzt.
Synthetische Öle und Wachse sind beispielsweise Silikonöle, Polyethylenglykole. Weitere geeignete hydrophobe Komponenten sind insbesondere Fettalkohole, vorzugsweise solche mit etwa 8 bis 22 Kohlenstoffatomen im Molekül wie Myristyl-, Cetyl-, Oleyl- und Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat.

Ihr Anteil an der Ölphase beträgt vorzugsweise, je nach Charakter und auch der Art der öllöslichen Emulgatoren, etwa 50 bis 95, insbesondere etwa 60 bis 90 Gew.-%.

Geeignete öllösliche Emugatoren sind insbesondere nichtionische wie die verschiedenen Fettalkoholethoxylate wie Lauryl-, Myristyl-, Cetyl- Oleyl-,Tridecyl-, Isotridecyl-, Cocosfett- und Talgfettalkoholethoxylate; jedoch können auch weitere an sich bekannte nichtionische öllösliche Emulgatoren verwendet werden.
Ihr Anteil in der Ölphase ist variabel und kann zwischen etwa 5 und etwa 50, ins besondere etwa 10 bis etwa 40 Gew.%,. der Ölphase betragen.

Als wasserlösliche Emulgatoren in der Wasserphase können insbesondere anionische Tenside verwendet werden.

Geeignete anionaktive Tenside im Rahmen der Erfindung sind in einer Menge von etwa 0,25 bis etwa 5 Gew.-%, vorzugsweise etwa 0,4 bis 2,5 Gew.-% der Gesamtzusammensetzung (einsatzfertige Emulsion) enthalten.

Dabei handelt es sich um solche vom Sulfat-, Sulfonat-, Carboxylat- und Alkylphosphat-Typ, vor allem natürlich diejenigen, die in Haarbehandlungsmitteln üblicherweise zum Einsatz gelangen, insbesondere die bekannten C₁₀-C₁₈-Alkylsulfate und die entsprechenden Ethersulfate, beispielsweise C₁₂-C₁₄-Alkylethersulfat, Laurylethersulfat, insbesondere mit 1 bis 4 Ethylenoxidgruppen im Molekül, Acylaminocarbonsäuren wie Lauroylsarkosinat und - glutamat, weiterhin Monoglycerid(ether)sulfate, Fettsäureamidsulfate, die durch Ethoxylierung und anschließende Sulfatierung von Fettsäurealkanolamiden erhalten werden, und deren Alkalisalze sowie Salze langkettiger Mono- und Dialkylphosphate, die milde, hautverträgliche Detergentien darstellen.

Im Rahmen der Erfindung weiterhin geeignete anionische Tenside sind α-Olefinsulfonate bzw. deren Salze und Alkalisalze von Sulfobernsteinsäurehalbestern, beispielsweise das Dinatriumsalz des Monooctylsulfosuccinats, und Alkalisalze langkettiger Monoalkylethoxysulfosuccinate.

Geeignete Tenside vom Carboxylat-Typ sind Alkylpolyethercarbonsäuren und deren Salze der Formel

R - (C₂H₄O)ₙ - O - CH₂ COOX,

worin R eine C₈-C₂₀-Alkylgruppe, vorzugsweise eine C₁₂-C₁₄-Alkylgruppe, n eine Zahl von 1 bis 20, vorzugsweise 2 bis 17, und X H oder vorzugsweise ein Kation der Gruppe Natrium, Kalium, Magnesium und Ammonium, das gegebenenfalls hydroxyalkylsubstituiert sein kann, bedeuten, sowie Alkylamidopolyethercarbonsäuren der allgemeinen Formel 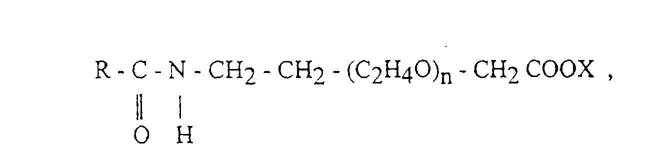 worin R und X die vorstehend angegebene Bedeutung haben und n insbesondere für eine Zahl von 1 bis 10, vorzugsweise 2,5 bis 5, steht.

Derartige Produkte sind seit längerem bekannt und im Handel, beispielsweise unter den Handelsnamen "AKYPO®" und "AKYPO-SOFT®".

Auch C₈-C₂₀-Acylisethionate können, allein oder im Gemisch mit anderen Tensiden, eingesetzt werden, ebenso Sulfofettsäuren und deren Ester.

Gegebenenfalls können auch amphotere bzw. zwitterionische Tenside als wasserlösliche Emulgatoren eingesetzt werden, insbesondere im Gemisch mit anionaktiven Tensiden, wobei die Gesamtmenge vorzugsweise bei etwa 0,25 bis 5 Gew.-% der Färbeemulsion liegen soll.

Als solche sind insbesondere die verschiedenen bekannten Betaine wie Fettsäureamidoalkylbetaine und Sulfobetaine, beispielsweise Laurylhydroxysulfobetain, zu nennen; auch langkettige Alkylaminosäuren wie Cocoaminoacetat, Cocoaminopropionat und Natriumcocoamphopropionat und -acetat haben sich als geeignet erwiesen.

Im einzelnen können Betaine der Struktur 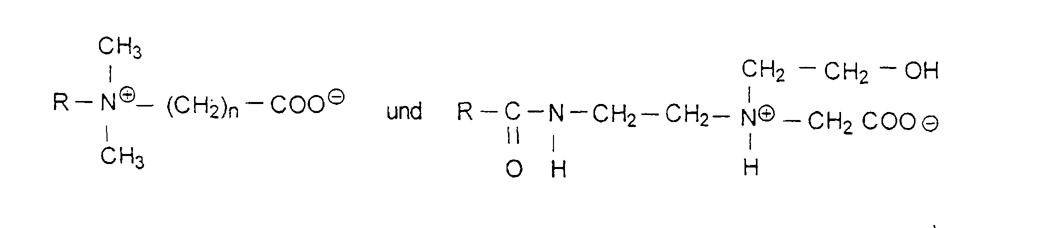 wobei R eine C₈-C₁₈-Alkylgruppe und n 1 bis 3 bedeuten,
Sulfobetaine der Struktur 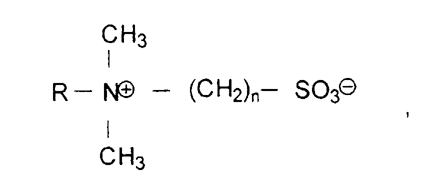 wobei R eine C₈-C₁₈-Alkylgruppe und n 1 bis 3 bedeuten,
und Amidoalkylbetaine der Struktur 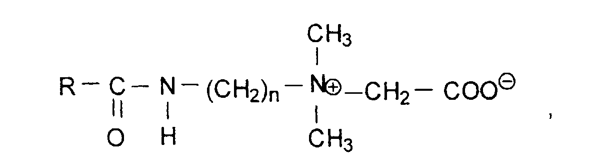 wobei R eine C₈-C₁₈-Alkylgruppe und n 1 bis 3 bedeuten, eingesetzt werden.

Auch die Verwendung nichtionischer wasserlöslicher Tenside, z.B. von C₈-C₁₈-Alkylpolyglucosiden mit einem Polymerisationsgrad von 1 bis 5, ist in den genannten Mengen alleine oder im Gemisch mit anionischen und/oder amphoteren bzw. zwitterionischen oberflächenaktiven Substanzen möglich.

Auch Aminoxide und Fettsäuremono- und -dialkanolamide sind einsetzbar.

Weitere geeignete Tenside sind auch kationische Tenside wie die bekannten quaternären Ammoniumverbindungen mit einer oder zwei Alkyl- bzw. Alkenylgruppen mit 10 bis 22 Kohlenstoffatomen im Molekül, insbesondere in einer Menge von 0,1 bis 7,5, vorzugsweise 0,25 bis 5, besonders bevorzugt 0,5 bis 2,5 Gew.-%, der Gesamtzusammensetzung.

Geeignete langkettige quaternäre Ammoniumverbindungen, die allein oder im Gemisch miteinander eingesetzt werden können, sind insbesondere Cetyltrimethylammoniumchlorid, Dimethyldicetylammoniumchlorid, Trimethylcetylammoniumbromid, Behenyltrimoniumchlorid, Stearyltrimethylammoniumchlorid, Dimethylstearylbenzylammoniumchlorid, Benzyltetradecyldimethylammoniumchlorid, Dimethyl- dihydriertes-Talgammoniumchlorid, Laurylpyridiniumchlorid, Lauryldimethylbenzylammoniumchlorid, Lauryltrimethylammoniumchlorid, Tris(oligooxyethyl)alkylammoniumphosphat, Cetylpyridiniumchlorid, etc. Grundsätzlich sind alle quaternären Ammoniumverbindungen geeignet, die im CTFA International Cosmetic Ingredient Dictionary unter der Bezeichnung " Quaternium" aufgeführt sind.

Die erfindungsgemäß hergestellte Haarfärbeemulsion enthält mindestens ein Oxidationsfarbstoffvorprodukt, zweckmäßigerweise ein Gemisch aus mindestens einer Entwickler- und mindestens einer Kupplersubstanz.

Diese sind an sich bekannt und beispielsweise in der Monographie von K. Schrader, Grundlagen und Rezepturen der Kosmetika, 2. Aufl. (1989), S. 784-799, beschrieben.

Beispielhafte Entwicklersubstanzen sind insbesondere 1,4-Diaminobenzol, 2,5-Diaminotoluol, Tetraaminopyrimidine, Triaminohydroxypyrimidine, 1,2,4-Triaminobenzol, 2-(2,5-Diaminophenyl)ethanol, 2-(2'-Hydroxyethylamino)-5-aminotoluol und 1-Amino-4-bis-(2'-hydroxyethyl)-aminobenzol bzw. deren wasserlösliche Salze; beispielhafte Kupplersubstanzen sind Resorcin, 2-Methylresorcin, 4-Chlorresorcin, 2-Amino-4-chlorphenol,4-(N-methyl)aminophenol, 2-Aminophenol, 3-Aminophenol, 1-Methyl-2-hydroxy-4-aminobenzol, 3-N,N-Dimethylaminophenol, 4-Amino-3-methylphenol, 5-Amino-2-methylphenol, 6-Amino-3-methylphenol, 3-Amino-2-methylamino-6-methoxypyridin, 2-Amino-3-hydroxypyridin, 4-Aminodiphenylamin, 4,4'-Diaminodophenylamin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, 1,3-Diaminotoluol, α-Naphthol, 1,4-Diamino-2-chlorbenzol, 4,6-Dichlorresorcin, 4-Hydroxy-1,2-methylendioxybenzol, 1,5-Dihydroxynaphthalin, 1,7-Dihydroxynaphthalin, 2,7-Dihydroxynaphthalin, 1-Hydroxynaphthalin, 4-Hydroxy-1,2-methylendioxybenzol, 2,4-Diamino-3-chlorphenol, und/oder 1-Methoxy-2-amino-4-(2'-hydroxyethylamino)-benzol, ohne daß diese beispielhafte Aufzählung Anspruch auf Vollständigkeit erheben könnte.

Entwickler- und Kupplersubstanzen sind vorzugsweise im Molverhältnis 1:3 bis 5:1, insbesondere etwa 1:1 und etwa 3:1, enthalten; ihr Anteil in der erfindungsgemäß hergestellten Farbemulsion kann jeweils etwa 0,1 bis etwa 5 Gew.-%, je nach gewünschter Färbung, betragen.

Diese Oxidationsfarbstoffvorprodukte sind zweckmäßigerweise bereits in der wäßrigen Phase enthalten; sie können jedoch auch, falls erwünscht, dem Fertigprodukt zusammen mit der Ölphase oder im Anschluß daran zugesetzt werden.

Die erfindungsgemäß hergestellten Zusammensetzungen können erwünschtenfalls auch sogenannte Nuanceure zur Feineinstellung des gewünschten Farbtones, insbesondere auch direktziehende Farbstoffe, enthalten.

Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäß hergestellten Haarfärbemittel können die in solchen Mitteln üblichen Grund- und Zusatzstoffe, Konditioniermittel, Stabilisatoren, Verdickungsmittel, Komplexbildner, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind.

Die erfindungsgemäß hergestellten Färbeemulsionen weisen vorzugsweise einen im alkalischen Bereich liegenden pH-Wert, insbesondere zwischen etwa 8 und etwa 12,5, vorzugsweise zwischen 8,5 und 11, auf.

Zur Applikation wird das erfindungsgemäß hergestellte Oxidationsfarbstoff-Vorprodukt mit einem Oxidationsmittel vermischt. Bevorzugtes Oxidationsmittel ist Wasserstoffperoxid, beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemitteln, d.h. nach Vermischung mit Peroxid, kann sowohl im schwach sauren, d.h. einem Bereich von 5,5 bis 6,9, im neutralen als auch im alkalischen Bereich, d.h. zwischen pH 7,1 und 10 liegen.

Im folgenden werden zwei Ausführungsbeispiele zur Erläuterung der Erfindung gegeben.

### Beispiel 1

22,5 g Cetylstearylalkohol, 67,5 g Paraffinöl und 10 g Oleth-5 werden unter inniger Vermischung bei 50°C geschmolzen und anschließend auf Raumtemperatur (20°C) abgekühlt.

Es wird eine homogene Paste erhalten (Ölphase).

In 80 g einer Lösung, enthaltend

| | |
|---|---|
| 0,5 g | Natriumlaurylsulfat, |
| 0,1 g | Ascorbinsäure, |
| 0,5 g | Ammoniumchlorid, |
| 0,2 g | Natriumsulfit, |
| 0,2 g | Tetranatrium-EDTA, |
| 10,0 g | 25%-iges Ammoniak, |
| 0,9 g | Weizenproteinhydrolysat, |
| 0,6 g | Panthenol, |
| 0,5 g | Hopfenextrakt, |
| 0,4 g | Parfumöl, |
| 0,55 g | p-Toluylendiaminsulfat, |
| 0,05 g | Resorcin, |
| 0,02 g | 4-Chlorresorcin, |
| 0,02 g | 3-Aminophenol, und |
| ad 80,00 g | Wasser |

wurden 20 g der Ölphase bei 25°C in einem Ultraturrax bei 9 500 U/min während 1 bis 2 Minuten eingerührt.

Das Produkt wies eine Viskosität von etwa 15 000 mPa.s, gemessen im Brookfield-Viskosimeter RVT bei 20°C, auf.

Es wurde eine stabile Färbemulsion erhalten, die nach Vermischung mit einer 6%-igen H₂O₂-Lösung im Verhältnis 1:1 eine Mittelblondfärbung ergab.

### Beispiel 2

169 g Oleylalkohol, 56 g Cetylstearylalkohol und 25 g Oleth-5 wurden bei 60°C zusammengeschmolzen und anschließend auf etwa 25°C abgekühlt.

25 g der so erhaltenen, pastenförmigen Ölphase wurde unter Rühren (etwa 10 000 U/min) bei etwa 20°C innerhalb von etwa 3 Minuten in 75 g einer Wasserphase der folgenden Zusammensetzung eingerührt:

| | |
|---|---|
| 0,50 g | Natriumlaurylsulfat, |
| 0,25 g | Cocoamidopropylbetain, |
| 0,05 g | Tetranatrium-EDTA, |
| 0,60 g | Panthenol, |
| 0,50 g | Ammoniumchlorid, |
| 1,00 g | Natriumsulfit, |
| 1,00 g | Weizenproteinhydrolysat, |
| 0,50 g | Roßkastanienextrakt, |
| 0,50 g | Ascorbinsäure, |
| 0,40 g | Parfum, |
| 0,40 g | p-Toluylendiaminsulfat, |
| 0,05 g | Resorcin, |
| 0,10 g | 3-Aminophenol, |
| 0,25 g | 4-Aminophenol, |
| 0,05 g | p-Amino-o-kresol, |
| ad 75,00 g | Wasser |

Es wird eine stabile Haarfärbeemulsion mit einer Viskosität von etwa 17 000 mPa.s bei 20°C (Brookfield-Viskosimeter RVT) erhalten, die nach der in Beispiel 1 beschriebenen Methode eine glänzende rotbraune Haarfärbung ergab.

Die Herstellung einer Färbeemulsion nach der bisher verwendeten Methode der Heißemulgierung aller Bestandteile betrug demgegenüber ein vielfaches der aufzuwendenden Zeitdauer.

Ein weiterer gravierender Vorteil des erfindungsgemäßen Verfahrens besteht darin, daß die Ölphase in größeren Mengen hergestellt und auf Vorrat zur kalten Herstellung weiterer Färbeemulsionen vorgehalten werden kann.

## Patentansprüche

1. Verfahren zur Herstellung von stabilen wäßrigen Haarfärbeemulsionen, enthaltend mindestens ein Oxidations-Haarfarbstoffvorprodukt, dadurch gekennzeichnet, daß zunächst eine mindestens einen öllöslichen Emulgator enthaltende homogene Ölphase durch Vermischen der Ölbestandteile bei erhöhter Temperatur zwischen 40 und 80°C hergestellt, und anschließend diese Ölphase mit einer wäßrigen Phase, die mindestens einen wasserlöslichen Emulgator enthält, unter Scherkraft bei 15 bis 30°C vermischt wird.

2. Verfahren nach Anspruch 1, dadurch gekennzeichnet, daß die Ölphase bei einer Temperatur von 45 bis 70°C hergestellt wird.

3. Verfahren nach Anspruch 1 oder 2, dadurch gekennzeichnet, daß die als Endprodukt erhaltene stabile wäßrige Haarfärbeemulsion eine Viskosität zwischen 2 500 und 25 000 mPa.s bei 20°C (Brookfield-Viskosimeter RVT) aufweist.

4. Verfahren nach Anspruch 3, dadurch gekennzeichnet, daß das Endprodukt eine Viskosität zwischen 5 000 und 20 000 mPa.s bei 20°C (Brookfield-Viskosimeter RVT) aufweist.

## Claims

1. Process for the preparation of stable aqueous hair dyeing emulsions, comprising at least one oxidation dyestuff precursor, characterized in first preparing a homogenous oily phase comprising at least one oil-soluble emulsifier, by mixing the oil components at a temperature between 40° and 80° C, and subsequently mixing this oily phase with an aqueous phase, comprising at least one water-soluble emulsifier under shearing force at a temperature between 15° and 30°C,

2. Process according to claim 1, wherein the oily phase is prepared at a temperature ranging from 45° to 70°C.

3. Process according to claims 1 or 2, wherein the stable aqueous hair dyeing emulsion has a viscosity between 2,500 and 25,000 mPa.s at 20°C (Brookfield-Viscosimeter RVT).

4. Process according to claim 3, wherein the hair dyeing emulsion has a viscosity between 5,000 and 20,000 mPa.s at 20°C (Brookfield-Viscosimeter RVT).

## Revendications

1. Procédé de préparation de teintures pour cheveux en émulsion aqueuse et stables, contenant au moins un précurseur de colorant d'oxydation capillaire, caractérisé en ce que l'on prépare d'abord une phase huileuse homogène contenant au moins un émulsifiant soluble dans l'huile, en mélangeant les composant huileux à une température élevée comprise entre 40 et 80 °C, puis en ce que l'on mélange cette phase huileuse avec une phase aqueuse qui contient au moins un émulsifiant soluble dans l'eau, sous l'effet d'une force de cisaillement et à une température comprise entre 15 et 30 °C.

2. Procédé selon la revendication 1, caractérisé en ce que la phase huileuse est préparée à une température comprise entre 45 et 70 °C.

3. Procédé selon la revendication 1 ou 2, caractérisé en ce que l'émulsion de teinture pour cheveux aqueuse et stable obtenue en tant que produit final présente une viscosité comprise entre 2500 et 25000 mPa.s à 20 °c (viscosimètre rotatif Brookfield RVT).

4. Procédé selon la revendication 3, caractérisé en ce que le produit final présente une viscosité comprise entre 5000 et 20000 mPa.s à 20 °C (viscosimètre rotatif Brookfield RVT).
